(19) Europäisches Patentamt
European Patent Office
Office européen des brevets

(11) **EP 2 349 459 B1**

(12) **EUROPEAN PATENT SPECIFICATION**

(45) Date of publication and mention
of the grant of the patent:
**19.07.2017 Bulletin 2017/29**

(51) Int Cl.:
***A61N 1/20*** *(2006.01)*   ***A61N 1/04*** *(2006.01)*

(21) Application number: **09791891.6**

(22) Date of filing: **25.08.2009**

(86) International application number:
**PCT/US2009/054903**

(87) International publication number:
**WO 2010/027792 (11.03.2010 Gazette 2010/10)**

(54) **TREATMENT OF SWEATING AND HYPERHIDROSIS**

BEHANDLUNG VON SCHWEISSAUSBRÜCHEN UND HYPERHIDROSE

TRAITEMENT DE LA TRANSPIRATION ET DE L'HYPERHIDROSE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **27.08.2008 US 199018**

(43) Date of publication of application:
**03.08.2011 Bulletin 2011/31**

(73) Proprietor: **Johnson & Johnson Consumer Inc.
Skillman, NJ 08558 (US)**

(72) Inventors:
• **FASSIH, Ali
Franklin Park
NJ 08823 (US)**
• **CHANTALAT, Jeanette
Pennington
NJ 08534 (US)**
• **LIU, Jue-Chen
Belle Mead
NJ 08502 (US)**
• **NEWBURGER, Joan, D.
West Trenton
NJ 08628 (US)**
• **SUN, Ying
Belle Mead
NJ 08502 (US)**

(74) Representative: **Kirsch, Susan Edith
Carpmaels & Ransford LLP
One Southampton Row
London WC1B 5HA (GB)**

(56) References cited:
**EP-A1- 0 277 314       WO-A-2009/045720
US-A1- 2004 167 461    US-A1- 2007 060 862
US-B2- 7 016 723**

**Description**

**FIELD OF THE INVENTION**

The present invention relates to devices and compositions for use in methods for the reduction of sweating.

**BACKGROUND OF THE INVENTION**

Electricity may be employed to provide stimulation to the skin tissue or to facilitate drug transport across the skin barrier. In electricity-assisted devices, an electric potential (voltage) is applied to the skin membrane, to facilitate electricity passage, or ionic drug transport through the skin, the latter is called transdermal iontophoretic drug delivery. In transdermal iontophoresis, an ionized drug migrates into the skin driven by an applied electric potential gradient. Anionic drugs are delivered into the skin under the cathode (negatively charged electrode), while cationic drugs are delivered under the anode (positively charged electrode). Iontophoresis enables enhanced as well as better control of permeation rate of the ionic species into the skin.

The most common design of an iontophoresis device includes a power source (e.g., a battery), an electric control mechanism, and two separate conductive electrodes. Each conductive electrode is in contact with a separate electrolyte composition (with or without an active agent). The electrolyte or ionic active composition is generally either an aqueous solution contained in a liquid chamber or a semi-solid. The assembly of the conductive electrode and electrolyte composition is often referred to as "an electrode assembly" or simply "an electrode." The two electrode assemblies are usually affixed to the skin separated by electric insulation between them.

Alternatively, the two electrode assemblies may be constructed into a single iontophoresis device with an electric insulating material built between the two electrode assemblies for electrical isolation to prevent shorting current. An example of such an iontophoresis device is disclosed in U.S. Patent No. 5,387,189.

In another variation of the common iontophoresis device designs, the electrolyte composition in one of the two electrode assemblies is eliminated, and the conductive electrode is placed directly in contact with the skin to complete the electric circuit. An example of such iontophoresis device is disclosed in U.S. Patent No. 6,385,487.

During a typical iontophoresis operation (mono-polar operation), one of the two electrodes (i.e., active electrode) drives the active agent into the skin. The other electrode (i.e., disperse electrode) serves to close the electrical circuit through the skin. Sometimes, a second active agent of opposite electric charge can be placed into electrolyte composition in contact with the second electrode, thus, being delivered into the skin under the second electrode. Alternatively, the electric polarity of the first and second electrodes can be reversed periodically to drive ionic species under both electrodes (bi-polar operation). A bi-polar iontophoresis device for transdermal drug delivery is disclosed U.S. Patent No. 4,406,658.

Iontophoretic devices are also used for the treatment of hyperhidrosis, excessive sweating typically of the palms of the hands, the soles of the feet, axilla, head, or face. It is estimated that approximately two percent of the population suffers from hyperhidrosis. Excessive sweating carries with it a social stigma, causing stress and anxiety in patients with the condition. Excessive sweating can lead to, or exacerbate dermatological disorders such as bacterial and fungal infections as well as play an inhibitory role in treatment of these conditions, as these microorganisms thrive in moist environments. In addition, sweating may promote the proliferation of odor causing microorganisms. Sweating also creates a nuisance and significant discomfort for patients wearing casts or with heavily bandaged wounds, as the moist environment may cause itching, odor, and infection.

Besides iontophoresis, conventional treatments for hyperhidrosis include the use of antiperspirants, aluminum chloride, botulinum toxin injections, and surgical procedures such as extrathoracic sympathectomy. Iontophoretic devices for the treatment of hyperhidrosis are described in example U.S. Patent Appln. Publication No. 2004/0167461 to Nitzan et al. and U.S. Patent No. 6,223,076 to Tapper. Nitzan et al. describe the use of a dermal patch that may be in the form of an article of clothing. The patch comprises an electrochemical cell having at least two electrodes positioned on one side of the dermal patch, the electrodes forming electrical contact with a skin portion of a subject. The patch is designed and configured for delivering an electric current through the skin and conductive fluid used in conjunction with the patch.

Tapper describes the delivery of an active ingredient, such as an antiperspirant, to a region of the human body using a device comprising a DC power source, a controller and a pair of electrodes. The electrodes are mounted in generally close proximity to one another and are separated by an insulating member. The device also comprises a pair of pads, each of which is positioned in adjacent contact with one of the electrodes. The electrodes are sized and arranged so that the tissue to be treated can extend across the insulating member and simultaneously contact both pads. The entire device, for example, fits within the armpit area. See also the Drionic Device, commercially available from General Medical Company (Los Angeles, CA), and the MD-1a Iontophoresis Unit commercially available from R.A. Fischer Company (North Ridge, CA).

Conventional iontophoretic devices like the above are less than optimal. They are inconvenient to use, and immobilize the patient during treatment. They also require the use of relatively high electric currents, around 18 milliamps, that are only manually adjustable, and which may, depending on the design, be directed through major portions of the body remote from the treatment area. They are also typically painful for the person undergoing treatment due to the high current. This is a particular problem in that treatment often requires several sessions over a period of weeks or months.

Devices, compositions and methods for sweat reduction have now been discovered that are simple to use, allow patient movement, and are relatively pain free.

In one embodiment, they employ a user-friendly garment, such as a glove or sock, containing a first electrode for contacting the treatment area. A second electrode is positioned on the skin nearby inside or outside the treatment area. The location of the second electrode is adjustable according to the desire and comfort of the user. The housing of electrodes within garments enables the user to tolerate extended treatment, which allows a lower current intensity to deliver a fixed dose of electricity, thus reducing unpleasant sensations inherent in typical iontophoresis devices. A power source connects the two electrodes and provides a low, adjustable electric current to the device. Importantly, the power source provides an electric current that is customizable for the patient, for example in current intensity and treatment duration. Optionally, a carrier such as water may be used to provide ionic communication between the first electrode, the second electrode, or both, and the skin.

In another embodiment, a composition comprising galvanic particulates comprised of two dissimilar conductive metals is used. Such galvanic particulates are described in WO 2009/045720. This composition may be applied directly to the skin in dry powder form, or as part of an anhydrous formulation. Upon use, the natural wetness of the skin activates an electrochemical reaction along the particulate surface, which generates low-level, sub-sensorial electricity. Optionally, an aqueous carrier may be added to the skin to enhance the electrochemical reaction. This device allows patients to passively undergo low-level iontophoresis treatment for longer durations without disruption of normal daily activities.

## SUMMARY OF THE INVENTION:

The invention is defined in claim 1.

In one aspect, the present invention features a device for sweat reduction treatment by application of electric current to a treatment area of the skin, which comprises: a) a garment comprising a first electrode adapted for contacting said treatment area; b) a second electrode adapted for contacting said treatment area or skin proximal to said treatment area; and c) a power delivery unit in electrical communication with said first and second electrodes, wherein said power deliver unit provides a customized dose of electricity to said treatment area.

The present invention also provides a device for sweat reduction by application of electric current to a palm of a human subject, which comprises: a) a glove comprising a first electrode adapted for contacting said palm; b) a second electrode adapted for location on the forearm adjacent said palm of said human subject; and c) a power delivery unit in electrical communication with said first and second electrodes, wherein said power delivery unit provides a customized dose of electricity to said palm.

The invention further provides a device for sweat reduction by application of electric current to a sole of a foot of a human subject, which comprises: a) a sock comprising a first electrode adapted for contacting said sole; b) a second electrode adapted for location on the leg adjacent said foot of said human subject; and c) a power delivery unit in electrical communication with said first and second electrodes, wherein said power delivery unit provides a customized dose of electricity to said sole.

The description also provides a method of sweat reduction by application of electric current to a treatment area of the skin, which comprises a) contacting said treatment area with a first electrode contained in a garment; b) contacting said treatment area or skin proximal to said treatment area with a second electrode, said first and second electrodes being in electrical communication with a power delivery unit; and c) providing a customized dose of electricity to said skin using said power delivery unit.

The present invention also provides a device for sweat reduction treatment by application of electric current to a treatment area of the skin, which comprises a garment comprising a composition comprising galvanic particulates including a first conductive material and a second conductive material, wherein both the first conductive material and the second conductive material are exposed on the surface of the particulates, the particle size of the particulates is from about 10 nanometers to about 100 micrometers, the second conductive material comprises from about 0.01 percent to about 10 percent, by weight, of the total weight of the particulates, and the difference between the standard potentials of the first conductive material and the second conductive material is at least about 0.2 V.

The invention further provides a device for sweat reduction treatment by application of electric current to a treatment area of the skin, which comprises a garment comprising a composition comprising galvanic particulates including a first conductive material and a second conductive material, wherein both the first conductive material and the second conductive material are exposed on the surface of the particulates, the particle size of the particulates is from about 10 nanometers to about 100 micrometers, the second conductive material comprises from about 0.01 percent to about 10 percent, by weight, of the total weight of the particulates, and the difference between the standard potentials of the first conductive material and the second conductive material is at least about 0.2V.

In another aspect, the present invention features a method of treating excessive sweating by application of electric current to a treatment area of the skin, which comprises topically applying a composition comprising galvanic particulates including a first conductive material and a second conductive material, wherein both the first conductive material and the second conductive material are exposed on the surface of the particulates, the particle size of the particulates is from about 10 nanometers to about 100 micrometers, the second conductive material comprises from about 0.01 percent to about 10 percent, by weight, of the total weight of the particulates, and the difference between the standard potentials of the first conductive material and the second conductive material is at least about 0.2 V.

**BRIEF DESCRIPTION OF THE DRAWINGS**

Figure 1 is a graph demonstrating the range of electricity dosage as a function of current intensity. The top and bottom curves illustrate maximum and minimum values, respectively. The intermediate line represents most preferred dosage value.

Figure 2 is a graph showing the treatment duration corresponding to the dosage curves of Figure 1.

Figure 3a an inside-out view of a glove housing an electrode according to the invention.

Figure 3b is a cross sectional view of the layers of the glove of Figure 3.

Figure 4a depicts an armband housing an electrode according to the invention.

Figure 4b is a cross sectional view of the armband of Figure 4a.

Figure 5 depicts a power delivery unit according to the invention.

Figure 6 depicts a device according to the invention on a person's arm during treatment.

**DETAILED DESCRIPTION OF THE INVENTION**

It is believed that one skilled in the art can, based upon the description herein, utilize the present invention to its fullest extent. The following specific embodiments are merely illustrative, and not limitative of the remainder of the disclosure in any way whatsoever.

Unless defined otherwise, all technical and scientific terms used herein have the same meaning as commonly understood by one of ordinary skill in the art to which the invention belongs. Unless otherwise indicated, a percentage refers to a percentage by weight (i.e., %(W/W)).

As used herein, "pharmaceutically-acceptable" means that the ingredients which the term describes are suitable for use in contact with the skin without undue toxicity, incompatibility, instability, irritation, allergic response, and the like.

As used herein, "safe and effective amount" means an amount of the ingredient or of the composition sufficient to provide the desired benefit at a desired level, but low enough to avoid serious side effects. The safe and effective amount of the ingredient or composition will vary with the area being treated, the age and skin type of the end user, the duration and nature of the treatment, the specific ingredient or composition employed, the particular cosmetically-acceptable carrier utilized, and like factors.

As used herein, the term "treatment" means the alleviation or elimination of symptoms, and/or cure, and/or prevention or inhibition of a disease or condition.

As used herein, "electronic communication" means the direct movement of electrons between two objects, for example the elements of the device (e.g., between the power source and the first and second electrodes).

As used herein, "ionic communication" means the movement of electrons between two objects, for example the elements of the device (e.g., the electrodes and carrier if present) and the skin, through the migration of ions as "electron movers" in contact with such objects (e.g., electrons pass between the electrodes and the skin via ionic transport of electrolytes (e.g., in the carrier) in contact with the electrode and the skin).

As used herein, "sweating" refers to the excretion of perspiration from the pores of the skin. Sweating is caused by conditions such as, but not limited to, non-pathological sweating, such as thermally-induced, exercise-induced, or stress-induced sweating; or pathological excessive sweating such as hyperhidrosis, including primary and secondary hyperhidrosis, as well as focal and generalized hyperhidrosis.

The device of the invention works by application of electric current to a treatment area. The treatment area may be, for example, the palm of a hand, the sole of a foot, the face, or the axilla.

In one embodiment, the device comprises electrodes that are affixed to the skin but separated from one another, such that all the electric current generated by the device travels through the skin to complete the electric circuit. In one embodiment the electrodes are affixed to the skin, and physically separated, but may be in contact with the same carrier (conductive solution) such that a fraction of the current flows through the skin, and the rest flows through the carrier.

The first electrode is adapted to contact the treatment area. The second electrode may be adapted to contact skin proximal to but outside the treatment area. Alternatively, both electrodes may be in contact with the treatment area, in which case both electrodes provide treatment. Advantageously, the location of the second electrode is adjustable, to provide extra flexibility for the user. For example, the first electrode may be adapted to contact the palm of a hand and the second electrode adapted to contact any part of the adjacent forearm. Alternatively, the first electrode may be adapted to contact the sole of a foot and the second electrode adapted to contact any part of the adjacent lower leg. A variety of configurations are possible.

In another embodiment, the electric current is generated by galvanic particles comprised of two dissimilar, electrically conductive materials that activate upon exposure to an aqueous environment. In this embodiment, the first and second electrodes are physically connected with each other as one particle. In one embodiment, the galvanic particles are applied to the skin in a dry powder form. In another embodiment, the galvanic particles are applied to the skin as part of an anhydrous or other formulation. In yet another embodiment, the galvanic particles can be applied to the skin as part of an anhydrous formulation, followed by addition of an aqueous carrier to enhance conductivity. Each metal component of the galvanic particle represents an electrode, with the generated current flowing in a one-compartment manner, wherein a fraction of the total current penetrates the skin.

Garment

The first electrode (and optionally the second electrode) is held in a garment. The garment may be fabricated into various shapes and sizes to fit the contours of various anatomical surfaces of the body. For example, it may be a glove, a sock, a hat, tights, a wrap, a cuff, a band such as an armband or leg band, a shoe, a shoe insert, a belt, a vest, or a shirt. In particular, the garment may be a glove or a sock for treatment of the palm of the hand or sole of the foot, respectively. For treatment of the axilla, the garment may consist of an underarm strap.

Preferably, the garment fits the treatment area snugly to provide good contact between the first electrode and the skin of the treatment area. In one embodiment, the first electrode contacts substantially all, that is, at least 80 percent, preferably 90 percent, of the surface area of the treatment area, for example upon wetting with a carrier. More preferably, the first electrode assembly contacts all, that is, 100 percent, of the surface area of the treatment area.

Preferably, the garment is fabricated without seams to avoid nonuniform contact with the skin that may result in uneven electric current distribution.

The second electrode may be held in a separate garment. For example, in an embodiment for treatment of the palm, the first electrode may be housed in a glove, while the second in a separate armband. Alternatively, the first and second electrodes may be combined into a single garment, such as a sleeve. Additionally, the first and second electrodes may constitute separate compartments within a single garment.

The garment may be made of a variety of materials that physically stabilize the electrode(s) and the carrier. The garment should be capable of absorbing carrier. Examples of materials for use in or as the garment include, but are not limited to: cotton-based gauze; non-woven pads made of rayon or a mixture of rayon, polyester and/or other polymer fibers; felts, woven fabrics, conductive nonwoven and woven materials, open-cell foam and sponge-like materials contained of polyurethane, polyester and/or other polymers; and cross-linked and noncross-linked gelling materials, such as polyacrylamide, polyvinyl alcohol, gelatin, hydroxymethyl cellulose, hydroxyethylcellulose, hydroxypropylcellulose, methylcellulose, and carboxymethyl cellulose.

In the most preferred embodiment, the material is relatively incompressible such that local variations in current density upon deformation are minimized.

Examples of further materials for use in or as the garment include, but are not limited to: hydrogels, cross-linked and non-cross-linked polymers; swellable polymers such as water-swollen cellulose derivatives (e.g., methylcellulose (MC), hydroxyethyl methylcellulose (HEMA), hydroxypropyl methylkcellulose (HPMC), ethylhydroxyethyl cellulose (EHEC), hydroxyethylcellulose (HEC), hydroxypropylcellulose (HPC), and carboxymethlcellulose (CMC) and their salts); polyvinyl alcohol (PVA); polyvinylpyrrolidone (PVP); polyethylene oxide (PEO); polymers prepared by monomers such as hydroxyethyl methacrylate (HEMA), hydroxyethoxyethyl methacrylate (HEEMA), hydroxydiethoxyethl methacrylate (HDEEMA), methyoxyethyl methacrylate (MEMA), methoxyethoxyethyl methacrylate (MEEMA), methyldiethoxyethyl methacrylate (MDEEMA), ethylene glycol dimethacrylate (EGDMA), n-vinyl- 2pyrrolidone (NVP), methacrylic acid (MA), and vinyl acetate (VAC); polycrylamide, polyacrylate polymers, cross-linked and non-cross-linked polyacrylic acids of various molecular weight and their salts (sodium, potassium, magnesium, calcium, aluminum, etc.); gelatin; gums and polysaccharides such as gum arabic, gum karaya, gum tragacanth, guar gum, gum benzoin, and alginic acid and their salts; polyethylene glycol (PEG); polypropylene glycol (PPG); and clays or other swellable minerals such as bentonite and montmorillonite.

In one embodiment, the material comprises a non-woven material. By "non-woven" is meant that the material, or a layer of the material, is comprised of fibers that are not woven into a fabric but rather are formed into a sheet, mat, or pad layer. The fibers can either be random (i.e., randomly aligned) or they can be carded (i.e., combed to be oriented in primarily one direction. Furthermore, the non-woven material can be composed of a combination of layers of random and carded fibers).

Non-woven materials may be comprised of a variety of natural and/or synthetic materials. By "natural" is meant that the materials are derived from plants, animals, insects, or byproducts of plants, animals, and insects. By "synthetic" is meant that the materials are obtained primarily from various man-made materials or from natural materials, which have been further altered.

Non-limiting examples of natural materials useful in the present invention are silk fibers, keratin fibers (such as wool fibers, camel hair fibers) and cellulosic fibers (such as wood pulp fibers, cotton fibers, hemp fibers, jute fibers, and flax fibers).

Examples of synthetic materials include, but are not limited to, those selected from the group containing acetate fibers, acrylic fibers, cellulose ester fibers, cotton fibers, modacrylic fibers, polyamide fibers, polyester fibers, polyolefin fibers, polyvinyl alcohol fibers, rayon fibers, polyurethane foam, and mixtures thereof.

Materials made from one or more of the natural and synthetic materials useful in the present invention can be obtained from a wide variety of commercial sources such as Freudenberg & Co. (Durham, NC USA), BBA Nonwovens (Nashville, TN USA), PGI Nonwovens (North Charleston, SC USA), Buckeye Technologies/Walkisoft (Memphis, TN USA), and Fort James Corporation (Deerfield, IL USA).

Methods of making non-woven materials are also well known in the art. Such methods include, but are not limited to, air-laying, water-laying, melt-blowing, spin-bonding, or carding processes. The resulting material, regardless of its method of production or composition, is then subjected to at least one of several types of bonding operations to anchor the individual fibers together to form a self-sustaining web. The non-woven substrate can be prepared by a variety of processes including hydro-entanglement, thermally bonding, and combinations of these processes. Moreover, the materials can have a single layer or multiple layers. In addition, a multi-layered material can include film layer(s) (e.g., aperture or non-aperture film layers) and other non-fibrous materials.

Strength, thickness, or firmness of the non-woven material may be a desirable attribute. This can be achieved, for example, by the addition of binding materials, such as wet strength resins, or the material may be made of polymer binder coatings, stable fibres, e.g. based on cotton, wool, linen and the like. Examples of wet strength resins include, but are not limited to, vinyl acetate-ethylene (VAE) and ethylene-vinyl chloride (EVCL) Airflex emulsions (Air Products, Lehigh, PA), Flexbond acrylic polymers (Air Products, Lehigh, PA), Rhoplex ST-954 acrylic binder (Rohm and Haas, Philadelphia, PA), and Ethylene-vinyl acetate (EVA) emulsion (DUR-O-SET® by National Starch Chemicals, Bridgewater, NJ). The amount of binding material in the substrate may range from about 5% to about 20%, by weight, of the substrate.

Non-woven materials of increased strength can also be obtained by using the so-called spunlace or hydro-entanglement technique. In this technique, the individual fibers are twisted together so that an acceptable strength or firmness is obtained without the need to use binding materials. The advantage of the latter technique is the excellent softness of the non-woven material.

Additives may also be added in order to increase the softness of the substrates. Examples of such additives include, but are not limited to, polyols such as glycerol, propylene glycol and polyethylene glycol, phthalate derivatives, citric esters, surfactants such as polyoxyethylene (20) sorbitanesters, and acetylated monoglycerides.

Waterproofing barriers may be incorporated into the garments to prevent leakage of water from the device, which may result in loss of efficacy. They may consist of impermeable films, such as polymer or latex films, or waterproofing treatments on any of the garments surfaces.

Sensory attributes may also be incorporated to the insoluble non-woven substrates. Examples of such sensory attributes include, but are not limited to color, texture, pattern, and embossing.

It is preferred that the second electrode be placed in proximity to the first electrode (i.e. treatment site) such that electrical current passing through vital organs of the body (e.g. heart) is minimized.

For treatment of the axilla, electrode arrangement will be designed such that current through internal organs is minimized.

Power Delivery Unit

The power delivery unit delivers and controls electricity flow to the electrodes. According to the invention, it comprises a logical method to deliver a customized, predetermined dose of electricity based on user input. This provides for more comfortable and effective treatment compared with conventional devices, which typically require manual adjustment of the current intensity.

Preferably, the power delivery unit is compact, portable, convenient, and allows patient functionality and mobility during use. In one embodiment the power delivery unit may be placed on a strap around the arm with leads protruding to the first and second electrodes. Alternatively, the power delivery unit may directly attach, with electrical connectivity, to one electrode with a lead connecting to the other electrode. In other embodiments, the power delivery unit may be worn on a leg strap, belt, wristband, necklace, headband, or other similar item.

The power delivery unit may provide conventional direct current (DC) or pulsed DC, such as that disclosed in U.S. Patent No. 5,042,975, alternating current (AC), or a combination. In one embodiment, the current density (current intensity per unit area of the skin) provided by the device in the present invention is generally less than about 0.5 mA/cm$^2$, such as less than about 0.1 mA/cm$^2$ or less than about 0.05 mA/cm$^2$. In one embodiment, the power delivery unit produces a voltage of from about 0.1 volts to about 9 volts, such as from about 1 to about 3 volts, such as about 1.5 volts. In one embodiment the power unit delivers up to but not exceeding 50 volts. The voltage may be stepped up electronically from a lower voltage. For example a 10-12 voltage may be stepped up to 50 volts.

In one embodiment, the power deliver unit is capable of automatically shutting off at the end of treatment.

The device may have built in safety features that monitor the electrical current delivered and automatically shut down the device. In one embodiment the device may have over current limit monitoring and protection with automatic shut down, with redundancy.

In one embodiment the device may include a built in electrode disconnection monitoring function with automated shut off. In one embodiment the device may include a load monitoring function to ensure that electrodes are properly attached. This function may automatically shut down the device in the case that a load is not detected.

In one embodiment, the power delivery unit is a battery (e.g., a rechargeable or disposable battery). In one embodiment, the battery is a disposable battery of small size, such as a button cell battery, suitable for a wearable patch or facial mask type adhesive device. Examples of suitable batteries include, but not limited to, button or coin batteries such as silver oxide, lithium, and zinc air batteries (which are typically used in small electronic devices). A zinc air battery is preferred because of its small size and high energy density, as well as its environmental friendliness. Examples of zinc air batteries include, but are not limited to, Energizer™ AC5 and AC10/230 (Eveready Battery Co. Inc., St. Louis, MO). Another preferred battery for the device is a flexible thin layer open liquid state electrochemical cell battery, such as a battery described in U.S. Patent No. 5,897,522.

Preferably, the electric current delivered by the device to the treatment area is at a low level, for example less than about 20 milliamps or 15 milliamps, more preferably less than about 12 milliamps. Use of the device is therefore relatively pain free. In one embodiment, the electric current delivered is up to 18 milliamps. To prevent burning and other forms of skin damage, it is preferred that the current density not exceed 1 milliamp/cm2.

In one embodiment, the device further comprises means for reversing the polarity of the first and second electrodes. Such means are advantageous in that pH changes arising from electrochemical reactions involving carrier are minimized. Upon operation of the device, the polarity may automatically switch once, or multiple times, depending on current intensity and duration. In addition, the current level may be higher during periods when the anode is at the non-treatment site, such that the cycle time dedicated to offsetting pH changes is minimized, thereby reducing overall treatment time.

In another embodiment, the polarity will not require reversal at all (e.g. 18 mA). For example, at higher current intensity, treatment will be run at one polarity and then terminated. It is preferred that the treatment site be the anode during this type of treatment.

It is preferred that the first electrode be the anode at the start of operation of the device for all treatments.

In one embodiment, power delivery unit delivers a continuously increasing amount of electricity followed by a continuously decreasing amount of electricity, optionally including one or more periods of constant electricity before and/or after the periods of increasing and decreasing electricity. For example, to minimize sensation, it is preferred that electric current be gradually ramped up from zero to a maximum value and then gradually ramped down again to zero. It is preferred that changes in polarity follow a gradual ramping cycle. Ramping is controlled by the power delivery unit. In one embodiment the total ramp time may be one or two minutes, and the current may be increased and decreased in this fashion over a plurality of cycles during treatment.

As a safety feature, the length of the two leads for each electrode may be varied to reduce the chance of incorrect connection by the user. Additionally, the wire connections may only fit one port on the power delivery unit to avoid misconnection.

The power unit may be designed to include a voltage monitoring. The rationale for this is to detect sudden changes in voltage, which may be undesirable. For example, a sudden drop in voltage may indicate compromised skin permeability during treatment (e.g. cut, blister formation). Sudden increased voltages may indicate loss of contact or less contact area and subsequent higher current density.

In an alternate embodiment, the device may be comprised of a galvanic couple of materials which generates electricity through electrochemical reactions initiated upon wetting of the system as disclosed in the US Patent Application Publication Nos. 2004/0267237, 2005/0004509, and 2005/0148996. For example zinc ink or another anodic ink may be screen-printed as the first electrode while silver-silver chloride ink or another cathodic ink may be printed as the second electrode. Electrodes may be in a one or two compartment arrangement and are activated upon wetting with carrier. Galvanic couples may also be created by conductive laminate materials.

Alternatively, the device may comprise electrodes in particulate form, for example particles comprising the first electrode and particles comprising the second electrode, or single particles comprising both the first electrode and second electrode, i.e., particles of the first electrode coated with second electrode material. Electrical current may be delivered to the skin using galvanic microparticles as described in the US Patent Application Publication No. 2007/0060862 A1.

Customized Dose

According to the invention, the electrical dose (mA min) delivered by the device may be customized and determined by an algorithm that uses the current intensity (mA) to calculate the appropriate dose for the user. Current intensity is selected by the user based on his tolerance level for the treatment and severity of condition. Based on this, the power delivery unit delivers a customized dose (z) to the user employing, for example, the following algorithm:

$$z = 360\ (1+x)$$

such that the benchmark dosage (360 mA min) is multiplied by a factor of 1+x to account for changes in current intensity (i). x varies with current intensity as:

$$x = (A-i/2)/6$$

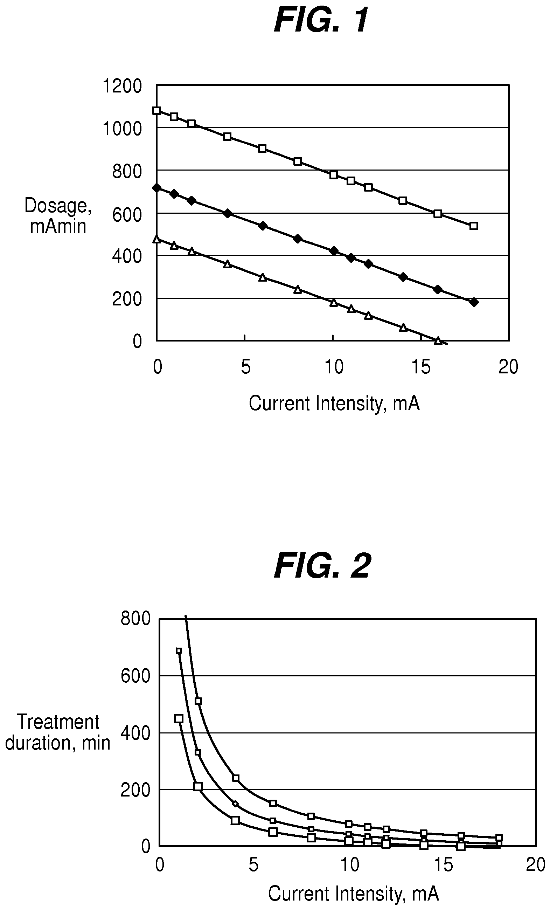

where A is a factor defined as follows. The claimed dose range is that which is comprised of the space between the dosage curves arising from the above equations with A=2.0 and A=24. Figure 1 shows the respective curves (triangles A=2, squares A=24). The most preferred dosage value is that obtained from a value of A=12 (diamonds). Figure 2 depicts a graph showing the treatment duration corresponding to the dosage curves of Figure 1.

Carrier

In one embodiment, the first electrode, the second electrode, or both are in ionic communication with a carrier containing an electrolyte. In the preferred embodiment, the first electrode and carrier for first electrode are separate from the second electrode and carrier for second electrode. The same or different carriers may be used with each electrode. The carrier may be a liquid (e.g., a solution, a suspension, or an emulsion that may be immobilized within the garment comprising an absorbent material such as gauze, cotton or non-woven pad made of synthetic or natural cellulose materials), a semi-solid (e.g., a gel, a cream, a lotion, microemulsion, or hydrogel), or a solid (e.g., a lyophilized foam composition that may be reconstituted by adding a liquid prior to use to form a gel) that during use is capable of conducting electricity from an electrode (e.g., the carrier may contains one or more electrolytes and water).

In one embodiment, the carrier (e.g., a liquid or semi-solid) is added to the first electrode by the user prior to or after applying the first electrode to the treatment area. For example, the carrier is added to a material for use in or as the garment (discussed below) comprising the first electrode. In one embodiment, the material is an absorbent material that can immobilize the carrier (such as gauze or non-woven pad) that contains or is in contact with the electrode (e.g., the first electrode is contained within or affixed to the absorbent material).

In one embodiment, the carrier is manufactured and placed in storage as a stable nonconductive composition (e.g., an anhydrous composition with negligible conductive ions). Prior to or during the use, as an activation step, water is mixed into the anhydrous composition to significantly increase its conductivity by enabling the passage of an electric current through the system. Examples of the carrier include, but are not limited to, purified water, tap water, distilled water, deionized water, skin creams, lotions, and polar solutions. Other examples of carriers include biological fluids or excretion such as sweat, skin moisture, interstitial fluid, intercellular fluid, wound exudates, blood, saliva, menstrual fluid, tears, urine, and vaginal fluid that exit the body and enter into the reservoir of the device. Examples of electrolytes include, but are not limited to, pharmaceutically acceptable organic and inorganic acids, bases, salts, buffers, peptides, polypeptides, proteins, nucleic acids, and/or other inorganic and organic compounds. Examples of inorganic salts include, but are not limited to, chloride salts (such as sodium chloride, potassium chloride, lithium chloride, calcium chloride, strontium chloride, magnesium chloride or other chloride salts), as well as salts of sodium, potassium, lithium, calcium, magnesium, strontium, fluoride, iodide, bromide. Examples of buffers include, but are not limited to, phosphates, citrates, acetates, lactates, and borates.

In one embodiment, the electrolyte is an active agent, or becomes an active agent after the passage of the electric current through the carrier. Examples of such electrolyte-active agents include, but are not limited to (anticholinergics) and other weak acid or weak base active agents.

In one embodiment, the carrier contains water. In a further embodiment, the carrier may also contain one or more organic solvents. Examples of organic solvents include, but are not limited to: dimethyl isosorbide; isopropylmyristate; surfactants of cationic, anionic and nonionic nature; vegetable oils; mineral oils; waxes; gums; synthetic and natural gelling agents; alkanols; glycols; and polyols.

In one embodiment, the carriers of the first and second electrode may be different. For example, the carrier for the first electrode may be purified water and the carrier for the second electrode may be a buffered solution.

Examples of glycols include, but are not limited to, glycerin, propylene glycol, butylene glycol, pentalene glycol, hexylene glycol, polyethylene glycol, polypropylene glycol, diethylene glycol, triethylene glycol, glycerol, and hexanetriol, and copolymers or mixtures thereof. Examples of alkanols include, but are not limited to, those having from about 2 carbon atoms to about 12 carbon atoms (e.g., from about 2 carbon atoms to about 4 carbon atoms), such as isopropanol and ethanol. Examples of polyols include, but are not limited to, those having from about 2 carbon atoms to about 15 carbon atoms (e.g., from about 2 carbon atoms to about 10 carbon atoms) such as propylene glycol.

The organic solvents may be present in the carrier in an amount, based upon the total weight of the carrier, of from about 1 percent to about 90 percent (e.g., from about 5 percent to about 50 percent). Water may be present in the carrier (prior to use) in an amount, based upon the total weight of the carrier, of from about 5 percent to about 95 percent (e.g., from about 50 percent to about 90 percent).

In one embodiment, the carrier is a nonconductive carrier such as an anhydrous composition that contains organic solvents that interact strongly when mixed with water during the application, resulting in the release of solvation heat to increase the temperature of the carrier and/or the power delivery unit, consequently increasing the electric current generated by either the battery or the galvanic power source. Examples of such organic solvents include, but are not limited to, glycerol, glycols (e.g., propylene glycol, butylenes glycol and ethylene glycol) and polyglycols (e.g., polyethylene glycols of various molecular weight, such as PEG400 and polypropylene glycols of various molecular weights).

The carrier may also contain: preservatives (such as cresol, chlorocresol, benzyl alcohol, methyl p-hydroxylbenzoate, propyl p-hydroxybenzoate, phenol, thimerosal, benzalkonium chloride, benzethonium chloride, and phenylmercuric nitrate); stabilizing agents or antioxidants (such as ascorbic acid, ascorbic acid esters, butylhydroxy anisole, butylhydroxy toluene, cysteine, N-acetylcysteine, sodium bisulfite, sodium metabisulfite, sodium formaldehydesulfoxylate, acetone sodium bisulfite, tocopherols, and nordihydroguaiaretic acid); chelating agents (such as ethylenediaminetetraacetic acid and its salts); buffers (such as acetic acid, citric acid, phosphoric acid, glutamic acid, and salts thereof); and tonicity adjusting agents (such as sodium chloride, sodium sulfate, dextrose and glycerin).

In one embodiment, the carrier is present in at least about 50%, such as at least about 75%, by weight, of the total weight of the garment prior to use. In another embodiment, (i) liquid carrier is present in less than about 10%, such as less than about 1%, by weight of the total weight of the garment (for example, the garment may not contain any carrier prior to use). In a further embodiment, the device is accompanied by instructions for the user to either (i) wet the garment prior to application or (ii) wet the skin with water and/or another liquid prior to or after application.

Electrodes

The electrodes may be reactive conductive electrodes or inert conductive electrodes. As used herein, a "reactive conductive electrode" is an electrode that undergoes a change in chemical composition during the chemical reactions occurring due to passage of electric current through the electrode. In one embodiment, the reactive conductive electrode is made of reactive materials such as metal halides (e.g., silver-silver chloride (Ag/AgCl), silver-silver bromide, and silver-silver iodide). In this case, the primary electrochemical reaction at the cathode surface is conversion of solid silver halide to metallic silver with little unwanted consumption of the oxidizing agents generated by the anode. The released halide ions may be subsequently oxidized to oxidizing agents, such as chloride ions to chlorine ($Cl_2$), hypochlorous acid (HClO), and hypochlorite ions ($ClO^-$), and iodide ions to iodine.

As used herein, an "inert conductive electrode" is an electrode that does not undergo a change in its chemical composition. In one embodiment, an inert conductive electrode is made of, or coated on the surface with, an inert materials such as noble metals (e.g., gold, platinum, gold-coated conductive metals), conductive carbon (e.g., glassy carbon or graphite), carbon-embedded polymers (e.g., conductive carbon silicone rubbers or conductive vinyl polymers), conductive carbon polymer foam or sponge, silver halide-coated silver (e.g., silver chloride-coated silver, silver bromide-coated silver, and silver iodide-coated silver), and corrosive resistant alloys such as stainless steel. Flexible and conformable conductive polyvinyl sheet electrode, manufactured by a printing or laminating method, is a preferred electrode material in the present invention.

Electrodes may also include conductive or metal plates, foils, meshs, or foams, or conductive non-woven or woven material embedded with thin metal wire such as stainless wire.

Electrodes may also be comprised of conductive woven or nonwoven materials. In one embodiment, the electrode may be a nonconductive woven or nonwoven specially knit with conductive fibers

In addition, electrodes may be composed of printed or sprayed conductive inks. Inks can be composed of conductive particles, such as carbon, silver, or stainless steel. The ink may contain a solvent and polymeric binder. Such printed or sprayed conductive films can be sprayed onto garment (i.e. nonwoven, socks), or other electrode supporting substrates (e.g. nitrile glove).

Electrodes may be molded to fit three dimensional surfaces of the body such as the underarm, or soles of the feet. This may be achieved either by molding conductive materials such as conductive polymers or metals, or by molding a nonconductive surface and laminating or spraying a conductive medium onto its surface to realize the three dimensional shape. For example, a nonconductive sole insert may be sprayed or screen printed with conductive ink, or laminated with conductive laminate to provide the conductive surface on the insole.

It is preferred that the surface area of the second electrode exceed that of the first electrode to reduce current density at the nontreatment surface, thereby reducing user sensation. Preferably, the surface area of the second electrode is more than two fold that of the first electrode. Alternately, in the case of lower intensity treatments where sensation may be minimal, it is desired that the second electrode be smaller than in high intensity treatment (less surface area). Also, it may be preferable that the second be equal to or smaller than the first electrode.

Electrode connections between the power delivery unit and electrodes may be completed using solder, conductive adhesive, lamination, electrical snaps, or other electrical connections. It is preferred that such connections be easy to attach for the everyday user. It is preferred that any conductive exposed wiring or connection junction to the electrodes be isolated from the carrier in order to avoid ion release into the carrier and eliminating undesired ionic delivery to the user, which may cause irritation. Electrical junctions may be isolated by coating with nonconductive or conductive polymer, covering with nonconductive or conductive laminate, adhesive, or tape.

In another embodiment, the electrical power may be transmitted wirelessly through RF technology, such that no electrical connectivity is needed between the power unit and electrodes.

In the most preferred embodiment, the electrodes may be arranged in such a way that all the current of the first electrode travels into the skin and returns through the second electrode (two compartment system). In another embodiment, a fraction of the current of the first electrode travels through the skin while the remaining fraction travels only through the carrier and/or solution (one compartment system) as disclosed in the US Patent Application Publication No. 2004/0267169.

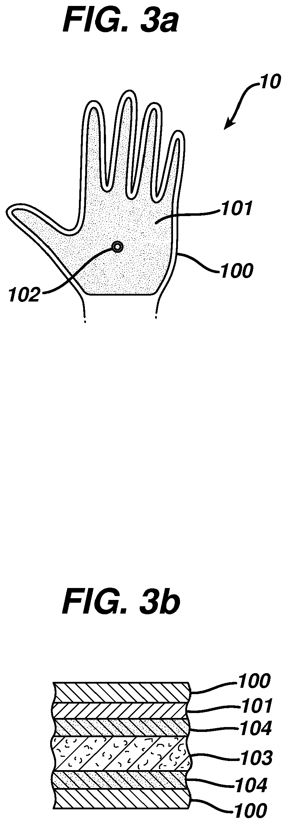

Referring to the drawings, Figure 3a shows an inside-out view of a garment **10** consisting of an outer glove **100** housing an electrode **101** on the palmar side. A conductive metallic snap **102** provides electrical connectivity between the electrode **101** and the outside of the glove.

Figure 3b shows a cross sectional view of the garment of Figure 3a in use. Hand **103** is in contact with wet absorbent material **104**. Electrode **101** is present on the palmar side of the hand only.

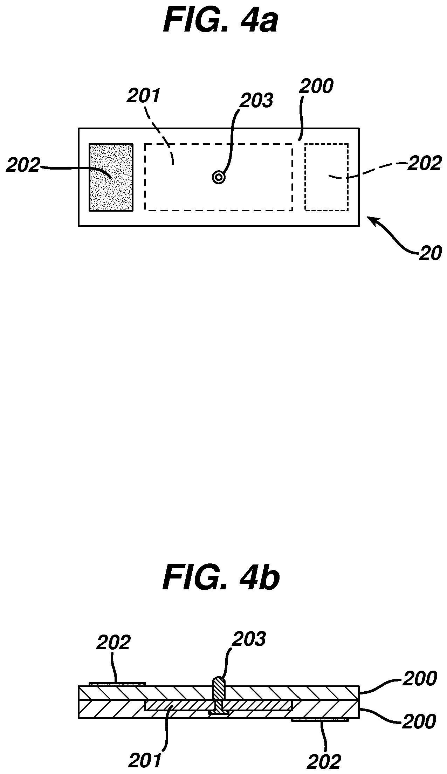

Figure 4a shows a top view of a multilayered armband garment **20** composed of an outer layer of absorbent material **200** enclosing an electrode **201**. The ends of the garment are fitted with Velcro **202** such that the user can affix the armband firmly to the arm. An electrical snap **203** provides electrical connectivity between the electrode **201** and the outside surface of the garment.

Figure 4b is a cross sectional view illustrating the layers of the garment in Figure 4a.

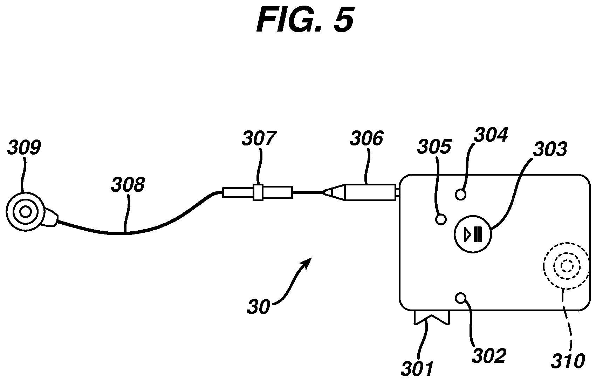

Figure 5 shows a power delivery unit **30** according to the invention. The power on/off switch **301** is toggled to activate or deactivate the device. An LED **302** indicates when the system is on. The treatment start/stop/pause button **303** is also shown. Two additional LED's represent diagnostic indicator **304** and "in-process" indicator **305**. The output plug **306** is connected to an inline fuse **307** and leads, via the positive electrode lead **308**, to a positive lead snap connector **309**. A negative lead port **310** is located on the bottom of the power delivery unit.

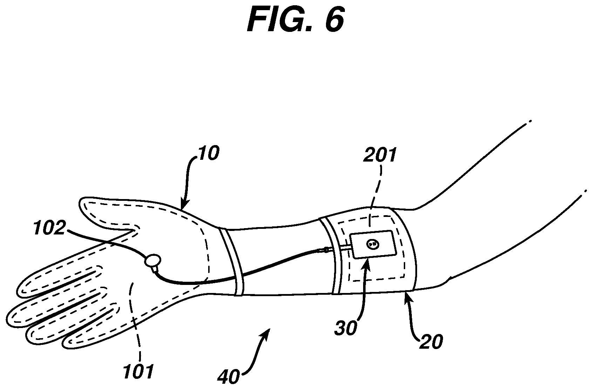

Figure 6 demonstrates a device **40** according to the invention as worn during treatment. Electrode **101**, which is in the shape of a palm and lies beneath the glove **10**, is shown as a dashed line. Similarly, electrode **201** contained in armband garment **20**, is shown as a dashed line. The negative snap **310** of the power delivery unit **30** is connected to the armband snap **203**.

Composition Comprising Galvanic Particulates

In an alternate embodiment, electric current can be applied to the skin by use of galvanic particulates which generate a low-level of electricity upon contact with an aqueous environment. Accordingly, a method for treating excessive sweating by topical application of a composition comprising such galvanic particulates is provided.

The composition may be applied to the treatment area in several forms including but not limited to: dry powder, powder formulated in an anhydrous or other formula such as a gel or cream for palms or feet; a gel, stick, or cream for the axilla; a cream, cleanser, or shampoo for the head and face. Optionally, users may separately apply to the treatment area an aqueous carrier such as lotion or gel, to enhance electrical conductivity of the composition.

In one embodiment, the galvanic particulates constitute 0.1% to 60% (by weight) of the composition. In a preferred embodiment, galvanic particulates constitute 0.5%-20% of the composition. In the most preferred embodiment, galvanic particulates constitute 1.0% to 5% (by weight) of the composition.

In one embodiment, the composition is used in conjunction with a garment of the kind described above but without the first and second electrodes (as the electric current is supplied by the composition). For example, users may apply the composition to the treatment area followed by covering with a garment to preserve the moist environment, intensify treatment, and/or prevent the galvanic particulates from unintentionally rubbing off. For the treatment of palmar sweating, a patient may apply the composition to the palm followed by application of a nitrile glove. Alternatively, the composition may be contained in the garment by incorporating galvanic particulates onto the surface of the garment. Methods of applying the galvanic particulates on the substrates include electrostatic spray coating, mechanical sieving, co-extrusion, adhesive spraying. The galvanic particulate device offers a convenient means of applying low-level, sub-sensory iontophoresis over an extended time.

Galvanic Particulates

The galvanic particulates of the present invention include a first conductive material and a second conductive material, wherein both the first conductive material and the second conductive material are exposed on the surface of the particulate. In one embodiment, the first conductive material is partially coated with the second conductive material.

In one embodiment, the galvanic particulates are produced by a coating method wherein the weight percentage of the second conductive material is from about 0.001% to about 20%, by weight, of the total weight of the particulate, such as from about 0.01 % to about 10%, by weight, of the total weight of the particulate. In one embodiment, the coating thickness of the second conductive material may vary from single atom up to hundreds of microns. In yet another embodiment, the surface of the galvanic particulate comprises from about 0.001 percent to about 99.99 percent such as from about 0.1 to about 99.9 percent of the second conductive material.

In one embodiment, the galvanic particulates are produced by a non-coating method (e.g., by sintering, printing or mechanical processing the first and the second conductive materials together to form the galvanic particulate) wherein the second conductive material comprises from about 0.1% to about 99.9%, by weight, of the total weight of the particulate, such as from about 10% to about 90%, of the total weight of the particulate.

In one embodiment, the galvanic particulates are fine enough that they can be suspended in the semi-solid compositions during storage. In a further embodiment, they are in flattened and/or elongated shapes. The advantages of flattened and elongated shapes of the galvanic particulates include a lower apparent density and, therefore, a better floating/suspending capability in the topical composition, as well as better coverage over the biological tissue, leading to a wider and/or deeper range of the galvanic current passing through the biological tissue (e.g., the skin or mucosa membrane). In one embodiment, the longest dimension of the galvanic particulates is at least twice (e.g., at least five times) the shortest dimension of such particulates.

The galvanic particulates may be of any shape, including but not limited to, spherical or non-spherical particles or elongated or flattened shapes (e.g., cylindrical, fibers or flakes).

In one embodiment, the average particle size of the galvanic particulates is from about 10 nanometers to about 500 micrometers, such as from about 100 nanometers to about 100 micrometers. As used herein, this is the maximum dimension of a particulate in at least one direction.

In one embodiment, the galvanic particulate comprises at least 90 percent, by weight, of conductive materials (e.g., the first conductive material and the second conductive material), such as at least 95 percent, by weight, or at least 99 percent, by weight, when a coating method is used for the production of the galvanic particulates.

Examples of combinations of first conductive materials/second conductive materials include (with a "/" sign representing an oxidized but essentially non-soluble form of the metal), but are not limited to, zinc-copper, zinc-copper/copper halide, zinc-copper/copper oxide, magnesium-copper, magnesium-copper/copper halide, zinc-silver, zinc-silver/silver oxide, zinc-silver/silver halide, zinc-silver/silver chloride, zinc-silver/silver bromide, zinc- silver/silver iodide, zinc-silver/silver fluoride, zinc-gold, zinc-carbon, magnesium-gold, magnesium-silver, magnesium-silver/silver oxide, magnesium-silver/silver halide, magnesium-silver/silver chloride, magnesium-silver/silver bromide, magnesium-silver/silver iodide, magnesium-silver/silver fluoride, magnesium-carbon, aluminum-copper, aluminum-gold, aluminum-silver, aluminum-silver/silver oxide, aluminum-silver/silver halide, aluminum-silver/silver chloride, aluminum-silver/silver bromide, aluminum-silver/silver iodide, aluminum-silver/silver fluoride, aluminum-carbon, copper-silver/silver halide, copper-silver/silver chloride, copper-silver/silver bromide, copper-silver/silver iodide, copper-silver/silver fluoride, iron-copper, iron-copper/copper oxide, copper-carbon iron-copper/copper halide, iron-silver, iron-silver/silver oxide, iron-silver/silver halide, iron- silver/silver chloride, iron-silver/silver bromide, iron-silver/silver iodide, iron-silver/silver fluoride, iron-gold, iron-conductive carbon, zinc-conductive carbon, copper-conductive carbon, magnesium-conductive carbon, and aluminum-carbon. Preferred first conductive metals include zinc, magnesium, aluminum, or their alloys thereof. The most preferred second metal is copper.

The first conductive material or second conductive material may also be alloys, particularly the first conductive material. Non-limiting examples of the alloys include alloys of zinc, iron, aluminum, magnesium, copper and manganese as the first conductive material and alloys of silver, copper, stainless steel and gold as second conductive material.

In one embodiment, the particulate, made of the first conductive material, is partially coated with several conductive materials, such as with a second and third conductive material. In a further embodiment, the particulate comprises at least 95 percent, by weight, of the first conductive material, the second conductive material, and the third conductive material. In one embodiment, the first conductive material is zinc, the second conductive material is copper, and the third conductive material is silver.

In one embodiment, the difference of the Standard Electrode Potentials (or simply, Standard Potential) of the first conductive material and the second conductive material is at least about 0.1volts, such as at least 0.2 volts. In one embodiment, the materials that make up the galvanic couple have a standard potential difference equal to or less than about 3 volts.

For example, for a galvanic couple comprised of metallic zinc and copper, the Standard Potential of zinc is -0.763V (Zn/Zn2+), and the Standard Potential of copper is +0.337 (Cu/Cu2+), the difference of the Standard Potential is therefore 1.100V for the zinc-copper galvanic couple. Similarly, for the magnesium-copper galvanic couple, Standard Potential of magnesium (Mg/Mg2+) is -2.363V, and the difference of the Standard Potential is therefore 2.700V. Additional examples of Standard Potential values of some materials suitable for galvanic couples are: Ag/Ag+: +0.799V, Ag/AgCl/Cl : 0.222V, and Pt/H2/H+: 0.000V. Pt may also be replaced by carbon or another conductive material. See, e.g., Physical Chemistry by Gordon M. Barrow, 4th Ed., McGraw-Hill Book Company, 1979, Page 626.

In one embodiment, the anodic metal may be aluminum, whose salts have been implicated in inhibiting sweating by mechanically obstructing sweat gland pores. For example, for a galvanic couple comprised of metallic aluminum and copper, the Standard Potential of aluminum is -1.676V (Al/Al3+), and the Standard Potential of copper is +0.337 (Cu/Cu2+), the difference of the Standard Potential is therefore 2.013 V for the zinc-copper galvanic couple. Upon activation of this galvanic particle within an aqueous environment, the galvanic particle will electrochemically release A13+ ions, which may mechanically clog the pores of the sweat glands, thereby reducing sweating.

Manufacture of Galvanic Particulates

In one embodiment, the conductive materials are combined (e.g., the second conductive material is deposited to the first conductive material) by chemical, electrochemical, physical or mechanical process (such as electroless deposition, electric plating, vacuum vapor deposition, arc spray, sintering, compacting, pressing, extrusion, printing, and granulation) conductive metal ink (e.g., with polymeric binders), and other known metal coating and powder processing methods commonly used in powder metallurgy, electronics and medical device manufacturing processes, such as the methods described in the book: "Asm Handbook Volume 7: Powder Metal Technologies and Applications" (by Asm International Handbook Committee, edited by Peter W. Lee, 1998, pages 31-109, 311-320).

In another embodiment, all the conductive materials are manufactured by chemical reduction processes (e.g., electroless deposition), sequentially or simultaneously, in the presence of reducing agent(s). Examples of reducing agents include phosphorous-containing reducing agents (e.g., a hypophosphite as described in US Patent Nos. 4,167,416 and 5,304,403), boron-containing reducing agents, and aldehyde- or ketone-containing reducing agents such as sodium tetrahydridoborate ($NaBH_4$) (e.g., as described in US 20050175649).

In one embodiment, the second conductive material is deposited or coated onto the first conductive material by physical deposition, such as spray coating, plasma coating, conductive ink coating, screen printing, dip coating, metals bonding, bombarding particulates under high pressure-high temperature, fluid bed processing, or vacuum deposition.

In one embodiment, the coating method is based on displacement chemical reaction, namely, contacting a particulate of the first conductive material (e.g., metallic zinc particle) with a solution containing a dissolved salt of the second conductive material (e.g. copper acetate, copper lactate, copper gluconate, or silver nitrate). In a further embodiment, the method includes flowing the solution over the particulate of the first conductive material (e.g., zinc powder) or through the packed powder of the first conductive material. In one embodiment, the salt solution is an aqueous solution. In another embodiment, the solution is contains an organic solvent, such as an alcohol, a glycol, glycerin or other commonly used solvents in pharmaceutical production to regulate the deposition rate of the second conductive material onto the surfaces of the first particulates, therefore controlling the activity of the galvanic particulates produced.

In another embodiment, the aforementioned displacement reaction for the formation of galvanic particulates may occur immediately prior to or during application by contacting the particulates of the first conductive material (e.g., metallic zinc particle) with a separately packaged solution containing a dissolved salt of the second conductive material (e.g. copper acetate, copper lactate, copper gluconate, or silver nitrate), By this means the galvanic particulates rapidly and spontaneously form in situ at the time of treatment. Under certain circumstances, in situ formation of galvanic particles may offer a more desirable commercial product for skin treatment as compared to pre-manufactured galvanic particulates described above.

In another embodiment, the galvanic particulates of the present invention may also be coated with other materials to protect the galvanic materials from degradation during storage (e.g., oxidation degradation from oxygen and moisture), or to modulate the electrochemical reactions and to control the electric current generate when in use. The exemplary coating materials over the galvanic material(s) are inorganic or organic polymers, natural or synthetic polymers, biodegradable or bioabsorbable polymers, silica, glass, various metal oxides (e.g., oxide of zinc, aluminum, magnesium, or titanium) and other inorganic salts of low solubility (e.g., zinc phosphate). The coating methods are known in the art of metallic powder processing and metal pigment productions, such as those described by U.S. Patent publications US 5,964,936; U.S. 5,993,526; US 7,172812; US 20060042509A1 and US 20070172438.

In one embodiment, the galvanic particulates are stored in anhydrous forms, e.g., as a dry powder or immobilized in a garment or fabric with binding agents, or as an essentially anhydrous non-conducting organic solvent composition (e.g., dissolved in polyethylene glycols, propylene glycol, glycerin, liquid silicone, and/or alcohol). In another embodiment, the galvanic particulates are embedded into the anhydrous carrier (e.g., inside a polymer) or coated onto a substrate (e.g., as a coating or in the coating layer of a healthcare product such as wound dressing or dental floss). In yet another embodiment, the galvanic particulates are encapsulated in compositions of microcapsules, liposomes, micelles, or embedded in the lipophilic phase of oil-in-water (O/W) or water-in-oil (W/O) types of emulsion systems (e.g., W/O lotion, W/O ointment, or O/W creams), as well as self-emulsifying compositions, in order to achieve self-life stability, retard the activation of the galvanic particulates, or prolong the action of galvanic particulates.

Compositions comprising galvanic particulates have great versatility and can be used in many forms such as creams, lotions, gels, shampoos, cleansers, powders, or in patches, bandages, masks, garments (such as undergarments, underwear, bras, shirts, pants, pantyhose, socks, head caps, facial masks, gloves, and mittens) or linens (such as towels, pillow covers or cases and bed sheets).

In one embodiment, the galvanic particulates are used to provide the intended therapeutic electric stimulation effects by applying the galvanic particulates directly to the target location of the body in need such a therapeutic treatment including but not limited to the axilla, the palms of the hand, sole of the feet, head, and face. Such therapeutic effects include, but are not limited to sweat reduction of the treated areas.

In one embodiment, the compositions contain a safe and effective amount of (i) the galvanic particulates and (ii) a pharmaceutically-acceptable carrier. Carriers may be selected as described above under the section entitled "Carrier."

Active Agents

In one embodiment, the device or composition of the invention also delivers one or more active agents into the skin. Such active agents include those either initially incorporated in the carrier, the composition comprising galvanic particulates, or electrochemically generated during use.

Examples of active agents for sweat reduction treatment include, but are not limited to: antiperspirants such as but not limited to aluminum salts such as aluminum chloride, aluminum chlorohydrate, and aluminum zirconium compounds such as aluminum zirconium tetrachlorohydrex gly. It is well known that aluminum based sweat reducing agents are irritating to the skin. Incorporation of galvanic particles into these compositions may counteract inflammation caused by aluminum salts, as galvanic particulates have been shown to exhibit anti-inflammatory activity in mammalian skin.

In another embodiment, the active agent for sweat reduction may include anticholinergic drugs; such as but not limited to oxybutynin, glycopyrrolate, propantheline bromide, and benzatropine; or Botox (botulinum neurotoxins), including Botox analogs, Botox active fragments, and natural Botox active metabolites.

In one embodiment, the carrier contains a safe and effective amount of the active agent, for example, from about 0.001 percent to about 20 percent, by weight, such as from about 0.01 percent to about 5 percent, by weight, of the carrier. The amount of the active agent in the carrier will depend on the active agent and/or the intended treatment area.

In one embodiment, the carrier contains metals such as metal ions or fine powders. Examples of such metals include, but are not limited to, gold, silver, copper, zinc.

In another embodiment, the composition containing the galvanic particulates further contain a safe and effective amount of the active agent, for example, from about 0.001 percent to about 20 percent, by weight, such as from about 0.01 percent to about 10 percent, by weight, of the composition.

The galvanic particulates can be combined with an active agent (such as antimicrobial agents, anti-inflammatory agents, and analgesic agents) to enhance or potentiate the biological or therapeutic effects of that active agent. In another embodiment, the galvanic particulates can also be combined with other substances to enhance or potentiate the activity of the galvanic particulates. Substances that can enhance or potentiate the activity of the galvanic particulates include, but are not limited to, organic solvents (such as alcohols, glycols, glycerin, polyethylene glycols and polypropylene glycol), surface active agents (such as nonionic surfactants, zwitterionic surfactants, anionic surfactants, cationic surfactants and polymeric surfactants), and water-soluble polymers. For example, the galvanic particulates of the present invention can form conjugates or composites with synthetic or natural polymers including by not limited to proteins, polysaccharides, hyaluronic acid of various molecular weight, hyaluronic acid analogs, polypeptides, and polyethylene glycols.

In one embodiment, the composition contains a chelator or chelating agent. Examples of chelators include, but are not limited to, amino acids such as glycine, lactoferrin, edetate, citrate, pentetate, tromethamine, sorbate, ascorbate, deferoxamine, derivatives thereof, and mixtures thereof. Other examples of chelators useful are disclosed in U.S. Pat. No. 5,487,884 and PCT Publication Nos. 91/16035 and 91/16034.

Other Ingredients

In one embodiment, the device or composition contains a plant extract as an active agent. Examples of plant extracts include, but are not limited to, feverfew, soy, glycine soja, oatmeal, what, aloe vera, cranberry, witch-hazel, alnus, arnica, artemisia capillaris, asiasarum root, birch, calendula, chamomile, cnidium, comfrey, fennel, galla rhois, hawthorn, houttuynia, hypericum, jujube, kiwi, licorice, magnolia, olive, peppermint, philodendron, salvia, sasa albomarginata, natural isoflavonoids, soy isoflavones, and natural essential oils.

In one embodiment, the device or composition contains ingredients to alleviate or prevent skin irritation and inflammation. In one embodiment these ingredients may be natural extracts, such as but not limited to feverfew, aloe, or chamomile. In one embodiment these ingredients may include topical steroids including corticosteroids, such as hydrocortisone. In one embodiment, the composition may include nonsteroidal anti-inflammatory agents.

In one embodiment, the device or composition contains a buffering agent such as citrate buffer, phosphate buffer, lactate buffer, gluconate buffer, or gelling agents, thickeners, or polymers.

In one embodiment, the device or composition contains a fragrance effective for reducing stress, calming, and/or affecting sleep such as lavender and chamomile.

Use

The devices and compositions of the invention are used for sweat reduction. Although applicants do not wish to be bound by theory, it is believed that the application of electric current to the skin plugs the pores of the treatment area, thereby preventing sweating. Alternatively, it may have a possible effect on the sweat gland by disturbing the electrical gradient that is thought to cause the movement of sweat along the sweat duct.

The device is used by placing the garment over the treatment area (optionally using a carrier such as water), such that the first electrode is in contact with the treatment area. Ideally, the garment will have a shape and size approximating the treatment area, as described herein, and first electrode will contact substantially all, preferably all of the treatment area. The second electrode is placed on the skin at a location within the treatment area or proximal to but outside of the treatment area. The current intensity is determined, and then the power delivery unit is activated and set at the desired current intensity, whereupon the power delivery device determines the correct treatment duration, after which the device preferably automatically turns off. Optionally, the polarity of the first and second electrodes are periodically reversed, such that electric current flows in one direction, then the opposite direction. Ideally, this device can be fully administered and operated by the user with no assistance needed from others.

In one treatment regimen the user may select from several current intensities based on his comfort requirements. The device automatically calculates the appropriate treatment duration of treatment corresponding to the user's desired treatment intensity and delivers the appropriate amount of electricity. The device may allow users the ability to choose from a continuous set of current intensities within a restricted range. Alternatively, the device may offer one or more predetermined treatment intensities (e.g. low, medium, or high).

Total electrical dose (e.g. charge, mA min) delivered to the skin may be prescribed by a physician and precisely monitored by the power delivery unit throughout treatment. In one embodiment, the physician may prescribe the total electrical dose (mA min), while the user chooses the desired current intensity of treatment (mA). In this embodiment the power delivery unit will monitor the length of treatment to achieve the desired dosage. Treatment duration is often longer than three minutes, preferably from 20 - 480 minutes. In another embodiment, the total dose and treatment duration are determined by the algorithm mentioned above.

In the case that a user or clinician wishes to pause treatment and continue shortly thereafter, the power delivery unit may include a treatment pause feature which ramps current to zero within a predetermined time interval. Treatment may resume, taking into account the paused time, as indicated by user or clinician. Current will ramp back to the treatment current and continue as programmed.

In one regimen, it may be desired that the user rub a thin layer of insulating material, such as silicone gel or petroleum jelly, over the skin prior to treatment. The rationale for this is that it has been shown that a thin layer of silicone gel enhances the efficacy of iontophoresis treatment for hyperhidrosis (Sato, K., Timm, D.E., Sato, F., Templeton, E.A., Meletiou, D.S., Toyomoto, T., Soos, G., Sato, S.K. Generation and Transit Pathway of H+ Ion is Critical for Inhibition of Palmar Sweating by Iontophoresis in Water. Journal of Applied Physiology. 75(5) 1993).

<u>Combination with other Energy Forms</u>

It may be desirable to combine use of the present device with other therapeutic forms of energy. For instance, in one embodiment the carrier may be heated to and maintained at, for example, about 35-45°C. This will result in sweat pore dilation during treatment and enhance treatment efficacy of the present invention. Additionally, warming may reduce unpleasant sensation of electric current. Heating may be achieved by building an ohmic, radio frequency, or infrared heating unit into the garments as well known in the art. The heating may also be generated from redox reactions unit such as disclosed in the US Patent No. 6,890,553 or from hydration heating when water is added to the system as disclosed in the US Patent No. 7,005,408. In another embodiment, it is desirable to cool the garment to alleviate sensation. This may be achieved by an electronic cooling unit, or by adding cooling salts to the solution. In yet another embodiment, it is desirable to couple electric treatment of present invention with ultrasound or laser treatment. In another embodiment, it is desirable to use a combination of the above-mentioned energies together.

<u>Examples</u>

The present invention will be further illustrated below by way of Examples, but the present invention is not limited thereto.

<u>Example 1:</u> The following examples illustrate use of the device to provide customized doses of electricity according to the invention using the algorithm described above. In each case, the user sets the current intensity, and the power delivery unit calculates the dose.

Case 1: User A selects current intensity of 12 mA (constant) - According to the algorithm, at 12 mA, the device delivers a predetermined dosage within the range of 120-720 mA min, with the preferred value at 360 mA min. Assuming a dosage level of 360 mA min, the power delivery unit delivers 12 mA for 30 minutes.

Case 2: User B selects a current intensity of 8 mA (constant) - According to the algorithm, at 8 mA, the device delivers a predetermined dosage within the range of 240-840 mA min, with the preferred value at 480 mA min. Assuming a dosage level of 480 mA min, the power delivery unit delivers 8 mA for 60 minutes.

Case 3: User C selects 10 mA (constant) for an early phase of treatment, then increases to 16 mA (constant) several weeks later. The user decides to change levels because his tolerance of the current increased - According to the algorithm, at the 10 mA initially chosen by the user, the dosage range is between 180-780 mA min, with the preferred value at 420 mA min. Assuming a dosage level of 420 mA min, the treatment duration is 42 minutes.

Several weeks later, when the user switches to a 16 mA current level, the dosage level changes to a range of 0-600 mA min, with the most preferred value of 240 mA min. Assuming 240 mA min, the treatment duration is 15 minutes.

Case 4: User D sets the current intensity to 6 mA and runs the treatment for 10 minutes, then increases the current intensity to 10 mA for the remainder of treatment because he can tolerate the treatment - Initially the current intensity is set to 6 mA. At this current intensity, the algorithm calls for a dosage range of 300-900 mA min, with the preferred dosage being 540 mA min. If 6 mA is delivered to the patient for 10 minutes, then 60 mA min of electrical dose is administered in that time.

If the user changes the current intensity to 10 mA, then a new dosage must be calculated for the remainder of treatment. At 10 mA, the algorithm calls for 180-780 mA min with the preferred value at 420 mA min. 60 maA min is delivered to the user during the first phase of treatment. The total remaining dosage (at 10 mA) is 360 mA min. To deliver 360 mA min at a 10 mA current level, the remaining duration of treatment is 36 minutes. Thus, the user undergoes 46 minutes of total treatment.

<u>Example 2: Galvanic Particulate Preparation Based on Displacement Chemistry</u>

(a) In Pure Aqueous Media: 0.1% copper coated zinc galvanic particulates were manufactured by electroless plating of copper onto zinc powder. 10g of <45-micron zinc powder was spread evenly onto a vacuum filter buchner funnel with a 0.22 micron filter. 5g of copper acetate solution was then poured evenly onto the zinc powder, and allowed to react for approximately 30 seconds. A suction was then applied to the filter until the filtrate was completely suctioned out. The resulting powder cake was then loosed, and 10 g of deionized water was added and then suctioned off. 10g of ethanol was then added to the powder under suction. The powder was then carefully removed from the filter system and allowed to dry in a desiccator.

(b) In Ethanol Containing Media: 0.1% copper coated zinc galvanic particulates were manufactured by electroless plating of copper onto zinc powder. 10g of <45-micron zinc powder was weighed into a glass jar. 0.61% w/w copper acetate was dissolved into 200 proof ethanol. The resulting copper solution is a faint blue color. 5g of copper acetate solution was then poured evenly onto the zinc powder, and allowed to react until the copper solution became clear. This reaction continued for approximately 48 hours at room temperature, when the solution turned clear. The composite was spread evenly onto a vacuum filter buchner funnel with a 0.22 micron filter. Vacuum suction was then applied to the filter until the filtrate was completely suctioned out. The resulting powder cake was then loosed, and 10 g of deionized water was added and then suctioned off. 10g of ethanol was then added to the powder under suction. The powder was then carefully removed from the filter system and allowed to dry in a desiccator.

(c) In Pure Aqueous Media: Approximately 0.1% copper coated magnesium galvanic particulates were manufactured by electroless plating of copper onto magnesium powder using the same method described in the Example 2 (a), except substituting zinc powder with magnesium powder.

(d) In Pure Aqueous Media: Approximately 0.1% iron coated magnesium galvanic particulates were manufactured by electroless plating of iron onto magnesium powder using same method described in the Example 2 (a), except substituting zinc powder with magnesium powder and the copper lactate solution with a ferrous chloride solution.

Example 3: Stick Compositions Containing Galvanic Particulates for Treatment of Excessive Sweating

Galvanic particles are made using the aforementioned displacement reaction between metallic zinc, metallic magnesium, or metallic aluminum particles and copper acetate to create zinc-copper, magnesium-copper, or aluminum-copper galvanic particles, respectively. Reaction conditions are controlled to deposit the desired amount of second conductive material onto the metallic particle. Stick formulations are made by incorporating the galvanic particulates into the formulations shown in Table 1.

The stick formulation is applied to the target skin once or twice per day and left on the skin following application. It is preferred that the formulation be reapplied after each washing.

**TABLE 1**

| Component | Formula 1 | Formula 2 | Formula 3 |
|---|---|---|---|
| Polybutene | 25.8%-30.7% | 15.8%-29.8% | 20.8%-28.8 |
| Phenyltrimethicone | 8.00% | 8.00% | 8.00% |
| Caprylic/ Capric Triglyceride | 3.00% | 3.00% | 3.00% |
| Octyldodecanol | 5.00% | 5.00% | 5.00% |
| Stearoyl inulin | 1.00% | 1.00% | 1.00% |
| Petrolatum | 0.50% | 0.50% | 0.50% |
| Butyrospermum Parkeii (Shea Butter) | 2.00% | 2.00% | 2.00% |
| Astrocaryum Muru-Muru Butter | 3.00% | 3.00% | 3.00% |
| Ozokerite | 7.00% | 7.00% | 7.00% |
| Pentaerythrityl Distearate | 2.00% | 2.00% | 2.00% |
| Carnauba (Copernicia Cerifera) Wax | 1.00% | 1.00% | 1.00% |
| Euphorbia (Candelilla) Cerifera Wax | 4.00% | 4.00% | 4.00% |
| Polyethylene | 4.00% | 4.00% | 4.00% |
| Hydrogenated Lanolin | 5.00% | 5.00% | 5.00% |
| Polyester-4 | 6.00% | 6.00% | 6.00% |
| Octinoxate | 7.50% | 7.50% | 7.50% |
| Titanium Dioxide and Hydrogenated Polyisobutene | 7.00% | 7.00% | 7.00% |

(continued)

| Component | Formula 1 | Formula 2 | Formula 3 |
|---|---|---|---|
| C10-30 Choolesterol/ Lanosterol Esters | 3.00% | 3.00% | 3.00% |
| Pentaerythrityl Di-t-butyl-Hydroxyhydrocinnamate | 0.10% | 0.10% | 0.10% |
| Methylparaben | 0.10% | 0.10% | 0.10% |
| Zinc-Copper Galvanic Particulate | 0.1%-5% | 0.00% | 0.00% |
| Aluminum-Copper Galvanic Particulate | 0.00% | 1%-10% | 0.00% |
| Magnesium-Copper Galvanic Particulate | 0.00% | 0.00% | 2%-10% |

The galvanic particulates may also be incorporated into a dry powder or anhydrous gel composition to be applied to the target skin area. Dry powder and anhydrous gel compositions, which can be used alone or together with a water containing formulation for skin applications, are well known in the art.

**Claims**

1. A device for the treatment of hyperhidrosis or for sweat reduction treatment by application of electric current to a treatment area of the skin, which comprises:

   a) a garment comprising a first electrode adapted for contacting said treatment area;
   b) a second electrode adapted for contacting the treatment area or skin proximal to the treatment area; and
   **characterised by**
   c) a power delivery unit in electrical communication with said first and second electrodes, wherein said power deliver unit provides a customized dose of electricity to said treatment area, wherein said power delivery unit delivers a continuously increasing amount of electricity followed by a first period of constant electricity, followed by a continuously decreasing amount of electricity, followed by a second period of constant electricity.

2. The device of claim 1, wherein said treatment area is the palm of a human subject, said garment comprises a glove and said second electrode can be adapted for location on the forearm adjacent said palm of said human subject.

3. The device of claim 1, wherein said treatment area is the sole of a foot of a human subject, said garment comprises a sock or shoe and said second electrode can be adapted for location on the leg adjacent said foot of said human subject

4. The device of any one of claims 1, 2 or 3, wherein said customized dose comprises a customized current intensity and customized duration of treatment.

5. The device of any one of claims 1, 2 or 3, wherein said power delivery unit is capable of automatically shutting off at the end of treatment.

6. The device of any one of claims 1, 2 or 3, further comprising one or more carriers in ionic communication with said first electrode, said second electrode, or both, wherein said carriers are in contact with the skin.

7. The device of claim 1, wherein said garment is selected from the group consisting of gloves, socks, hats, tights, wraps, cuffs, armbands, leg bands, shoes, shoe inserts, belts, underarm straps, vests, and shirts.

8. The device of any one of claims 1, 2, or 3, wherein said power delivery unit provides an electric current of less than about 18 milliamps.

9. The device of any one of claims 1, 2, or 3, wherein said garment comprises a material comprising a non-woven.

10. The device of any one of claims 1, 2, or 3, wherein said power delivery unit is a direct current power delivery unit.

11. The device of any one of claims 1, 2, or 3, further comprising means for reversing the polarity of said first and second electrodes.

12. The device of any one of claims 1, 2, or 3, wherein said customized dose is calculated using the algorithm:

$$z = 360 (1+x)$$

wherein z is said customized dose, x is defined as:

$$x = (A-i/2)/6$$

i is current intensity, and A is 2 to 24.

**Patentansprüche**

1. Vorrichtung zur Behandlung von Hyperhidrose oder zur Schweißreduktionsbehandlung durch Anlegen von elektrischem Strom an einen Behandlungsbereich der Haut, welche umfasst:

   a) ein Kleidungsstück, das eine erste Elektrode umfasst, die dafür ausgelegt ist, den Behandlungsbereich zu kontaktieren;
   b) eine zweite Elektrode, die dafür ausgelegt ist, den Behandlungsbereich oder Haut in der Nähe des Behandlungsbereichs zu kontaktieren; und
   **gekennzeichnet durch**
   c) eine Stromzuführungseinheit, die mit der ersten und der zweiten Elektrode in elektrischer Kommunikation steht, wobei die Stromzuführungseinheit dem Behandlungsbereich eine individuell angepasste Dosis von elektrischem Strom liefert, wobei die Stromzuführungseinheit eine stetig zunehmende Menge an Strom liefert, gefolgt von einer ersten Periode konstanten Stroms, gefolgt von einer stetig abnehmenden Menge an Strom, gefolgt von einer zweiten Periode konstanten Stroms.

2. Vorrichtung nach Anspruch 1, wobei der Behandlungsbereich der Handteller eines menschlichen Probanden ist, das Kleidungsstück einen Handschuh umfasst und die zweite Elektrode für eine Anordnung auf dem Unterarm, welcher dem Handteller des menschlichen Probanden benachbart ist, angepasst werden kann.

3. Vorrichtung nach Anspruch 1, wobei der Behandlungsbereich die Sohle eines Fußes eines menschlichen Probanden ist, das Kleidungsstück eine Socke oder ein Schuh ist und die zweite Elektrode für eine Anordnung auf dem Bein, welches dem Fuß des menschlichen Probanden benachbart ist, angepasst werden kann.

4. Vorrichtung nach einem der Ansprüche 1, 2 oder 3, wobei die individuell angepasste Dosis eine individuell angepasste Stromstärke und eine individuell angepasste Behandlungsdauer umfasst.

5. Vorrichtung nach einem der Ansprüche 1, 2 oder 3, wobei die Stromzuführungseinheit in der Lage ist, am Ende der Behandlung automatisch auszuschalten.

6. Vorrichtung nach einem der Ansprüche 1, 2 oder 3, welche ferner einen oder mehrere Träger umfasst, die in ionischer Kommunikation mit der ersten Elektrode, der zweiten Elektrode oder beiden stehen, wobei sich die Träger in Kontakt mit der Haut befinden.

7. Vorrichtung nach Anspruch 1, wobei das Kleidungsstück aus der Gruppe ausgewählt ist, welche aus Handschuhen, Socken, Hüten, Bodys, Umhängen, Manschetten, Armbändern, Fußbändern, Schuhen, Schuheinlagen, Gürteln, Unterarmgurten, Westen und Hemden besteht.

8. Vorrichtung nach einem der Ansprüche 1, 2 oder 3, wobei die Stromzuführungseinheit einen elektrischen Strom von weniger als etwa 18 Milliampere liefert.

9. Vorrichtung nach einem der Ansprüche 1, 2 oder 3, wobei das Kleidungsstück ein Material umfasst, das ein Faservlies umfasst.

10. Vorrichtung nach einem der Ansprüche 1, 2 oder 3, wobei die Stromzuführungseinheit eine Gleichstrom-Zuführungseinheit ist.

11. Vorrichtung nach einem der Ansprüche 1, 2 oder 3, welche ferner Mittel zum Umkehren der Polarität der ersten und der zweiten Elektrode umfasst.

12. Vorrichtung nach einem der Ansprüche 1, 2 oder 3, wobei die individuell angepasste Dosis mithilfe des Algorithmus

$$z = 360 \ (1+x)$$

berechnet wird, wobei z die individuell angepasste Dosis ist, x als

$$x = (A-i/2)/6$$

definiert ist, i die Stromstärke ist und A 2 bis 24 ist.

**Revendications**

1. Dispositif pour le traitement de l'hyperhidrose ou pour un traitement de réduction de la sueur par l'application d'un courant électrique à une zone de traitement de la peau, qui comprend :

   a) un vêtement comprenant une première électrode adaptée pour venir en contact avec ladite zone de traitement ;
   b) une seconde électrode adaptée pour venir en contact avec la zone de traitement ou la peau à proximité de la zone de traitement ; et
   **caractérisé par**
   c) une unité de distribution de puissance en communication électrique avec lesdites première et seconde électrodes, dans lequel ladite unité de distribution de puissance fournit une dose personnalisée d'électricité à ladite zone de traitement, dans lequel ladite unité de distribution de puissance distribue une quantité d'électricité augmentant en continu suivie par une première période d'électricité constante, suivie par une quantité d'électricité diminuant en continu, suivie par une seconde période d'électricité constante.

2. Dispositif selon la revendication 1, dans lequel ladite zone de traitement est la paume d'un sujet humain, ledit vêtement comprend un gant et ladite seconde électrode peut être adaptée pour un emplacement sur l'avant-bras adjacent à ladite paume dudit sujet humain.

3. Dispositif selon la revendication 1, dans lequel ladite zone de traitement est la plante d'un pied d'un sujet humain, ledit vêtement comprend une chaussette ou une chaussure et ladite seconde électrode peut être adaptée pour un emplacement sur la jambe adjacente audit pied dudit sujet humain.

4. Dispositif selon l'une des revendications 1, 2 ou 3, dans lequel ladite dose personnalisée comprend une intensité de courant personnalisée et une durée de traitement personnalisée.

5. Dispositif selon l'une quelconque des revendications 1, 2 ou 3, dans lequel ladite unité de distribution de puissance est capable de s'éteindre automatiquement à la fin du traitement.

6. Dispositif selon l'une quelconque des revendications 1, 2 ou 3, comprenant en outre un ou plusieurs supports en communication ionique avec ladite première électrode, ladite seconde électrode, ou les deux, dans lequel lesdits supports sont en contact avec la peau.

7. Dispositif selon la revendication 1, dans lequel ledit vêtement est choisi dans le groupe constitué des gants, des chaussettes, des chapeaux, des collants, des écharpes, des manches, des brassards, des bandes de jambe, des chaussures, des semelles, des ceintures, des lanières d'aisselle, des vestes, et des chemises.

**8.** Dispositif selon l'une quelconque des revendications 1, 2, ou 3, dans lequel ladite unité de distribution de puissance fournit un courant électrique inférieur à environ 18 milliampères.

**9.** Dispositif selon l'une quelconque des revendications 1, 2 ou 3, dans lequel ledit vêtement comprend un matériau comprenant un non-tissé.

**10.** Dispositif selon l'une quelconque des revendications 1, 2, ou 3, dans lequel ladite unité de distribution de puissance est une unité de distribution de puissance à courant continu.

**11.** Dispositif selon l'une quelconque des revendications 1, 2, ou 3, comprenant en outre un moyen pour inverser la polarité desdites première et seconde électrodes.

**12.** Dispositif selon l'une quelconque des revendications 1, 2, ou 3, dans lequel ladite dose personnalisée est calculée en utilisant l'algorithme :

$$z = 360(1 + x)$$

dans lequel z est ladite dose personnalisée, x est défini commue :

$$x = (A-i/2)/6$$

i est l'intensité de courant, et A est 2 à 24.

## FIG. 1

## FIG. 2

# FIG. 3a

# FIG. 3b

# FIG. 4a

# FIG. 4b

# FIG. 5

# FIG. 6

EP 2 349 459 B1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5387189 A
- US 6385487 B
- US 4406658 A
- US 20040167461 A, Nitzan
- US 6223076 B, Tapper
- WO 2009045720 A
- US 5042975 A
- US 5897522 A
- US 20040267237 A
- US 20050004509 A
- US 20050148996 A
- US 20070060862 A1
- US 20040267169 A

- US 4167416 A
- US 5304403 A
- US 20050175649 A
- US 5964936 A
- US 5993526 A
- US 7172812 B
- US 20060042509 A1
- US 20070172438 A
- US 5487884 A
- US 9116035 W
- US 9116034 W
- US 6890553 B
- US 7005408 B

**Non-patent literature cited in the description**

- Physical Chemistry. McGraw-Hill Book Company, 1979, 626
- Asm Handbook Volume 7: Powder Metal Technologies and Applications. Asm International Handbook Committee, 1998, vol. 7, 31-109, 311-320

- **WATER.** *Journal of Applied Physiology,* 1993, vol. 75 (5

26